# EUROPEAN PATENT SPECIFICATION

(11) **EP 1 478 312 B1**
(45) Date of publication and mention of the grant of the patent: **13.09.2006**
(21) Application number: 03737396.6
(22) Date of filing: 23.01.2003
(51) Int. Cl.: A61F 7/00, A45D 26/00, F25D 3/00

(54) **PERSONAL CARE SYSTEM WITH A PERSONAL CARE DEVICE AND A COOLING DEVICE**
SYSTEM FÜR DIE PERSÖNLICHE PFLEGE MIT HYGIENEVORRICHTUNG UND KÜHLVORRICHTUNG
SYSTEME DE SOINS PERSONNELS AVEC DISPOSITIF DE SOINS PERSONNELS ET DISPOSITIF DE REFROIDISSEMENT

(30) Priority: 06.02.2002 EP 02100111
(43) Date of publication of application: 24.11.2004
(73) Proprietor: Koninklijke Philips Electronics N.V., 5621 BA Eindhoven (NL)
(72) Inventor: FERTNER, Rembert, NL-5656 AA Eindhoven (NL); SAFARIK, Paval, NL-5656 AA Eindhoven (NL)
(74) Representative: van der Veer, Johannis Leendert
(86) International application number: PCT/IB2003/000185
(87) International publication number: WO 2003/065948

(56) References cited:
- EP-A- 0 348 862
- WO-A-00/76363
- FR-A- 2 709 932

## Description

The invention relates to a personal care system with a personal care device and a cooling device, which cooling device has a housing of plastics material, in which housing a cooling agent is contained, and which cooling device has a heat-conducting device.

The invention further relates to a cooling device for a personal care system, which cooling device has a housing of plastics material, in which housing a cooling agent is contained, and which cooling device has a heat-conducting device.

A personal care system as briefly described above, namely a depilation system, and a cooling device as briefly described above for this depilation system, are known from patent document WO 00/76363 A1. In the known solution, the heat-conducting device, which is made of aluminum, has a first portion that is accommodated in the housing of the cooling device and that is in heat-conducting connection with the cooling agent, which first portion is connected to a second portion of the heat-conducting device that is designed to be of a substantially sleeve-like form and is of an oval configuration, the second portion of sleeve-like form being closed off at one end by a sleeve floor, which sleeve floor forms a third portion of the heat-conducting device that is intended for cooling an area to be cooled of a person's body. In the known design, the second portion of sleeve-like form is provided in the area of the outer wall of the sleeve with groove-like depressions that are closed on themselves like rings and that receive strip-like projections from the housing that are also closed on themselves like rings, which strip-like projections are produced by an injection-molding process. The cross-sectional configuration of the groove-like depressions is U-shaped in this case, with the side-walls converging slightly in each depression looking in the direction of the bottom wall of the depression. Due to the sleeve-like form of the second portion of the heat-conducting device in the known solution, the elasticity that exists in the area of the second portion is sufficiently high to ensure that, in spite of the differences in temperature that occur in a cooling device of this kind, there is a good seal between the plastics housing and the second portion of the aluminum heat-conducting device, thus preventing any unwanted escape of the cooling agent contained in the housing.

Investigations have shown that where the second portion of a heat-conducting device, which second portion passes through a housing, is differently configured, the solution known from patent WO 00/76363 A1 will unfortunately not meet requirements because, when the second portion of a heat-conducting device is of metal and is, for example, of a more or less plate-like configuration and is surrounded by a housing of plastics material, unwanted leaks may occur when a solution known from patent WO 00/76363 A1 is implemented, due to the considerable difference in the coefficients of thermal expansion.

An object that the invention has set for itself is to overcome the difficulties outlined above and to produce an improved personal care system and an improved cooling device.

To achieve the object stated above, features according to the invention are provided in a personal care system according to the invention such that a personal care system according to the invention can be characterized in the manner stated below.

A personal care system with a personal care device and a cooling device, which cooling device has a housing of plastics material, in which housing a cooling agent is contained, and which cooling device has a heat-conducting device, wherein the heat-conducting device has a first portion that is accommodated in the housing of the cooling device and that is in heat-conducting connection with the cooling agent, a second portion that passes through the housing in a direction of passage and a third portion that is intended for cooling an area to be cooled of a body, wherein there is provided between the second portion of the heat-conducting device and the area of the housing surrounding the second portion of the heat-conducting device at least one combination, extending transversely to the direction of passage, of at least one groove-like depression and at least one strip-like projection, and wherein the at least one groove-like depression in the at least one combination is defined by two side-walls of which at least one side-wall has a configuration such that a portion of the strip-like projection hooks behind it.

To achieve the object detailed above, features according to the invention are provided in a cooling device according to the invention such that a cooling device according to the invention can be characterized in the manner stated below.

A cooling device for a personal care system, which cooling device has a housing of plastics material, in which housing a cooling agent is contained, and which cooling device has a heat-conducting device, wherein the heat-conducting device has a first portion that is accommodated in the housing and that is in heat-conducting connection with the cooling agent, a second portion that passes through the housing in a direction of passage and a third portion that is intended for cooling an area to be cooled of a body, wherein there is provided between the second portion of the heat-conducting device and the area of the housing surrounding the second portion of the heat-conducting device at least one combination, extending transversely to the direction of passage, of at least one groove-like depression and at least one strip-like projection, and wherein the at least one groove-like depression in the least one combination is defined by two side-walls of which at least one side-wall has a configuration such that a portion of the strip-like projection hooks behind it.

By providing the features according to the invention, it is possible, in a manner which is structurally simple and easy for manufacture, to produce an advantageous design in which it is always ensured, even when the temperature differentials to which a cooling device according to the invention is exposed are high, that a satisfactory seal will be maintained in the cooling device in that area in which the second portion of the heat-conducting device passes through the housing of the cooling device. The satisfactory sealing action exists chiefly as a result of the fact that, if the cooling device is cooled down, as happens if it is placed in the freezer compartment of a refrigerator or in a freezer cabinet, in which case the plastics housing of the cooling device, and hence the second portion of the heat-conducting device too, will contract to a greater degree than the metal heat-conducting device, then each portion of a strip-like projection that hooks behind a depression side-wall will press against the depression side-wall that it hooks behind with a relatively high force and will thereby ensure a safe and reliable seal, and it also exists as a result of the fact that, when the cooling device warms up, as happens when the personal care system and hence the cooling device is being used, at which time the plastics housing of the cooling device will expand to a greater degree than the metal heat-conducting device, and hence than the second portion of the heat-conducting device, each strip-like projection will press against the bottom wall of a groove-like depression, and also against the transitional areas between a groove-like depression and the adjoining outer face of the part of the cooling device that has the groove-like depression, with a relatively high force, thus once again ensuring by this means that a satisfactory seal exists.

In a personal care system according to the invention or in a cooling device according to the invention it is possible for only one depression side-wall to have a configuration such that a portion of a strip-like projection hooks behind it. It has, however, proved particularly advantageous if both side-walls of at least one groove-like depression have a configuration such that two portions of an associated strip-like projection hook behind them. A design of this kind ensures particularly high reliability with regard to a good sealing action.

In a personal care system according to the invention or in a cooling device according to the invention, the groove-like depression may be arcuate in cross-section. It has proved particularly advantageous if the at least one groove-like depression is of dovetail shape. A design of this kind has been found to be especially advantageous with regard to a seal that is as good as possible.

In a personal care system according to the invention and in a cooling device according to the invention, the groove-like depressions may also extend only over those areas of the housing or of the second portion of the heat-conducting device that are of relatively large size transversely to the direction of passage. What has proved highly advantageous, however, is for the features specified in claim 4 and claim 10 to be provided in addition in the respective cases. What this means is that the at least one groove-like depression extends over the entire extent of the second portion of the heat-conducting device, or over the entire extent of the area of the housing surrounding said second portion, in a position oriented transversely to the direction of passage, as a result of which it is ensured that there is a satisfactory seal along the entire extent.

In a personal care system according to the invention and in a cooling device according to the invention, it has proved advantageous if the features specified in claims 5 and 11 respectively are provided. A design of this kind has been found to be advantageous in practice. However, it should be mentioned that it may also be advantageous if the at least one strip-like projection is provided on the housing of the cooling device and the associated groove-like depression is provided in the second portion of the heat-conducting device.

In a personal care system according to the invention and in a cooling device according to the invention, it has proved especially advantageous if the heat-conducting device comprises at least one extruded member of shaped profile. This design has the great advantage that the heat-conducting device can be produced with particular ease by an extrusion process.

These and other aspects of the invention are apparent from and will be elucidated with reference to the embodiments described hereinafter, although the invention is not limited to these embodiments.

In the drawings:
Fig. 1 is an oblique view from above of a personal care system according to the invention, with a personal care device and with a cooling device according to the invention.
Fig. 2 is a view similar to Fig. 1 showing the cooling device of the personal care system of Fig. 1.
Fig. 3 is a view of the personal care system of Fig. 1 in section, taken on line III-III in Fig. 1.
Fig. 4 is a detail view, in section, of the area of the cooling device of the personal care system of Figs. 1 and 3 defined by circle IV in Fig. 3.
Fig. 5 is a view in section showing part of a heat-conducting device of a cooling device in a second embodiment of the invention.

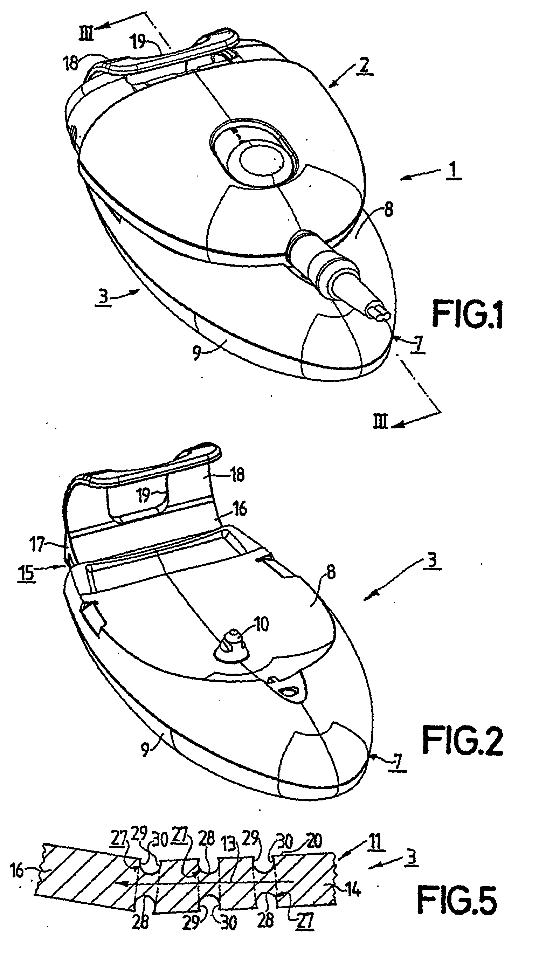

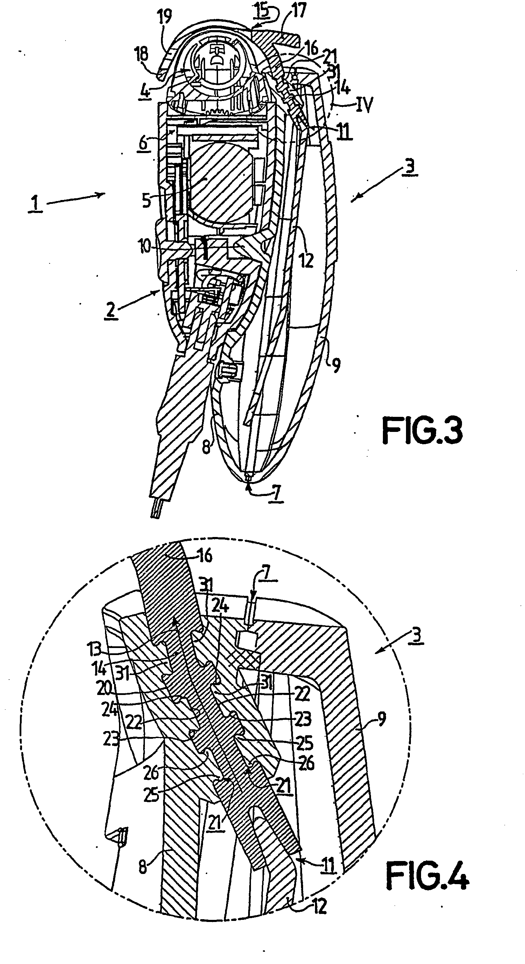

Shown in Figs. 1 and 3 is a personal care system. The personal care system 1 comprises a personal care device 2 and a cooling device 3. The personal care device 2 is a depilation device 2 by means of which hairs can be extracted from areas of a person's body. To allow the hairs to be extracted, the depilation device 2 has a set 4 of depilation disks that are situated next to one another and driven in rotation, which disks can be driven by a motor 5 via a gearbox 6. The design of depilation devices 2 of this kind has been known for quite some time and is immaterial in the present connection and for this reason no further details of the design of the depilation device 2 will be given here.

The cooling device 3, which is shown on its own in Fig. 2 and of which a detail is shown in Fig.4, has a housing 7. The housing 7 comprises two dishes 8 and 9 that are made of plastics material and are connected together with a liquid-tight seal by a welded joint in the plastics material. Contained in the housing 7 is a cooling agent that comprises mainly water plus suitable additives. In the area of the first housing dish 8, the cooling device 3 has a snap-in projection 10 by means of which the cooling device 3 can be connected to the depilation device 2, thus enabling the cooling device 3 and the depilation device 2 to form a working combination suitable for practical operation, namely the personal care system 1.

The cooling device 3 also has a heat-conducting device 11. The heat-conducting device 11 comprises a portion 12 that is completely accommodated in the housing 7 and is in thermally conductive connection with the cooling agent, a second portion 14 that passes through the housing 7, that is to say through dish 8 of the housing in Fig.4, in a direction of passage indicated by an arrow 13, and a third portion 15 that is intended to cool an area to be cooled of a person's body. In the present case, the heat-conducting device 11 comprises two extruded shaped profiles of aluminum, with the first extruded shaped profile forming the first portion 12 and the second extruded shaped profile forming the second portion 14 and the third portion 15. The two shaped profiles are connected together by a compressed joint formed by a compression operation. The first portion 12 is designed to be substantially plate-like and slightly angled so that its configuration is matched as closely as possible to the shape of the housing 7. It should be mentioned that the first portion 12 may also be formed by a stamped part. The third portion 15 comprises a connecting portion 16 that joins up in one piece with the second portion 14, a main cooling portion 15 that projects from the connecting portion 16 and a substantially U-shaped extra cooling portion 18 that is connected both to the connecting portion 16 and to a main cooling portion 17 and that surrounds an entry opening 19 that is provided to allow the hairs to enter and reach the set 4 of depilation disks.

The heat-conducting device 11 is provided, at its second portion 14, with six groove-like depressions 21 that extend transversely to the direction of passage 13 and that open onto the outer surface 20 of the second portion 14. The six groove-like depressions 21 are of a substantially dovetailed shape in the present case. Each of the six depressions 21 is defined by a bottom wall 22 and two side-walls 23 and 24, which side-walls 23 and 24 merge on the one hand with the bottom wall 22 and on the other with the outer surface 20 of the second portion 14. The configuration of the side-walls 23 and 24 of the depressions is, in a particularly advantageous manner, such that portions 25 and 26 of the housing 7 hook behind the side-walls 23 and 24, which portions 25 and 26, like the entire housing dish 8, were produced by injection molding them around the second portion 14 of the heat-conducting device 11. The injection molding around the second portion 14 of the heat-conducting device 11 forms strip-like projections 31, the portions 25 and 26 of which are intended and designed to hook behind the side-walls 23 and 24 of the depressions.

Because of the form as described above of the groove-like depressions 21, of the side-walls 23 and 24 of the depressions and of the strip-like projections 31, what is advantageously achieved is that a satisfactory seal will always be ensured, thus reliably preventing any unwanted escape of cooling agent from the cooling device 3 in the area of the second portion 14 that passes through the housing 7 of the cooling device 3, that is to say even when the cooling device 3 is exposed to high temperature differentials, which happens in practice because the cooling device is placed, for example, in a freezer cabinet or in the freezer compartment of a refrigerator to cool it down and then, when in use, is surrounded by the prevailing ambient air, which may be at high temperature.

A second portion 14 of the heat-conducting device 11 of a cooling device 3 is shown diagrammatically in Fig. 5. In this solution, there are three groove-like depressions 27 provided and these are formed to be closed on themselves like rings. In this case too, the groove-like depressions 27 are each defined by a bottom wall 28 and two side-walls 29 and 30, with walls 28, 29 and 30 together being of an arcuate configuration and the configuration of the side-walls 29 and 30 of the depressions being such that portions of the strip-like projections (not shown in this case) on the housing of the cooling device 3 hook behind these side-walls 29 and 30.

In the case of the solutions described above by reference to Figs. 1 to 5, the groove-like depressions 21 and 27 are provided in the second portion 14 of the heat-conducting device 11 and the strip-like projections 31 are provided on the housing 7 of the cooling device 3. It is, however, also possible for the strip-like projections. to be provided on the heat-conducting device and the groove-like depressions to be provided in the housing, the advantage of which is that there is no weakening of the material of the heat-conducting device caused by depressions.

Described above by reference to Figs. 1 to 4 is a personal care system 1 that comprises a depilation device 2 and a cooling device 3. The provisions according to the invention may however be made, with advantage, not only in a personal care system with a depilation device but also in a personal care system employing a massaging unit or a circulation-stimulating unit having a skin-treatment appliance having needle-like or pin-like treatment members, in which personal care system a cooling device may equally well be used, with advantage, to reduce the sensation of pain.

## Claims

1. A personal care system (1) with a personal care device (2) and with a cooling device (3), which cooling device (3) has a housing (7) of plastics material, in which housing (7) a cooling agent is contained, and which cooling device (3) has a heat-conducting device (11), wherein the heat-conducting device (11) has a first portion (12) that is accommodated in the housing (7) and is in thermally conductive connection with the cooling agent, a second portion (14) that passes through the housing (7) in a direction of passage (13) and a third portion (15) that is intended for cooling an area to be cooled of a body, **characterized in that** there is provided between the second portion (14) of the heat-conducting device (11) and the area of the housing (7) surrounding the second portion (14) of the heat-conducting device (11) at least one combination, extending transversely to the direction of passage (13), of at least one groove-like depression (21) and at least one strip-like projection (31), and **in that** the at least one groove-like depression (21; 27) in the at least one combination is defined by two side-walls (23, 24; 29, 30) of which at least one side-wall (23, 24; 29, 30) is of a configuration such that a portion (25, 26) of the strip-like projection (31) hooks behind it.

2. A personal care system (1) as claimed in claim 1, wherein the configuration of the two side-walls (23, 24; 29, 30) of the depressions is such that two portions (25, 26) of the strip-like projection (31) hook behind them.

3. A personal care system (1) as claimed in claim 1, wherein the at least one groove-like depression (21) is of dovetail shape.

4. A personal care system (1) as claimed in claim 1, wherein at least one combination comprises a groove-like depression (27) that is closed on itself like a ring and a strip-like projection that is closed on itself like a ring.

5. A personal care system (1) as claimed in claim 1, wherein, in the at least one combination, the at least one groove-like depression (21; 27) is provided in the second portion (14) of the heat-conducting device (11) and the at least one strip-like projection (31) is provided on the area of the housing (7) surrounding the second portion (14) of the heat-conducting device (11).

6. A personal care system (1) as claimed in claim 1, wherein the heat-conducting device (11) comprises at least one extruded part of shaped profile made of aluminum.

7. A cooling device (3) for a personal care system (1), which cooling device (3) has a housing (7) of plastics material, in which housing (7) a cooling agent is contained, and which cooling device (3) has a heat-conducting device (11), wherein the heat-conducting device (11) has a first portion (12) that is accommodated in the housing (7) and is in thermally conductive connection with the cooling agent, a second portion (14) that passes through the housing (7) in a direction of passage (13) and a third portion (15) that is intended for cooling an area to be cooled of a body, **characterized in that** there is provided between the second portion (14) of the heat-conducting device (11) and the area of the housing (7) surrounding the second portion (14) of the heat-conducting device (11) at least one combination, extending transversely to the direction of passage (13), of at least one groove-like depression (21) and at least one strip-like projection (31), and **in that** the at least one groove-like depression (21; 27) in the at least one combination is defined by two side-walls (23, 24; 29, 30) of which at least one side-wall (23, 24; 29, 30) is of a configuration such that a portion (25, 26) of the strip-like projection (31) hooks behind it.

8. A cooling device (3) as claimed in claim 7, wherein the configuration of the side-walls (23, 24; 29, 30) of the depressions is such that two portions (25, 26) of the strip-like projection (31) hook behind them.

9. A cooling device (3) as claimed in claim 8, wherein the at least one groove-like depression (21) is of dovetail shape.

10. A cooling device (3) as claimed in claim 7, wherein at least one combination comprises a groove-like depression (27) that is closed on itself like a ring and a strip-like projection that is closed on itself like a ring.

11. A cooling device (3) as claimed in claim 7, wherein, in the at least one combination, the at least one groove-like depression (21; 27) is provided in the second portion (14) of the heat-conducting device (11) and the at least one strip-like projection (31) is provided on the area of the housing (7) surrounding the second portion (14) of the heat-conducting device (11).

12. A cooling device (3) as claimed in claim 7, wherein the heat-conducting device (11) comprises at least one extruded part of shaped profile made of aluminum.

## Patentansprüche

1. Körperpflegesystem (1) mit einer Körperpflegeeinrichtung (2) und einer Kühlvorrichtung (3), wobei die Kühlvorrichtung (3) ein Gehäuse (7) aus Kunststoff aufweist, in dem Gehäuse (7) ein Kühlmittel enthalten ist und die Kühlvorrichtung (3) eine Wärmeleiteinrichtung (11) aufweist, und wobei die Wärmeleiteinrichtung (11) einen in dem Gehäuse (7) untergebrachten und mit dem Kühlmittel in Wärmeleitverbindung stehenden ersten Abschnitt (12), einen durch das Gehäuse in einer Durchgangsrichtung (13) hindurchgehenden zweiten Abschnitt (14) und einen zum Kühlen eines zu kühlenden Bereiches eines Körpers vorgesehenen dritten Abschnitt (15) aufweist, **dadurch gekennzeichnet, dass** zwischen dem zweiten Abschnitt (14) der Wärmeleiteinrichtung (11) und dem den zweiten Abschnitt (14) der Wärmeleiteinrichtung (11) umgebenden Bereich des Gehäuses (7) mindestens eine quer zur Durchgangsrichtung (13) verlaufende Kombination aus mindestens einer rillenförmigen Vertiefung (21) und mindestens einer leistenförmigen Erhebung (31) vorgesehen ist, und dass
die mindestens eine rillenförmige Vertiefung (21; 27) in der mindestens einen Kombination von zwei Vertiefungsseitenwänden (23, 24; 29, 30) begrenzt ist, von denen mindestens eine Vertiefungsseitenwand (23, 24; 29, 30) so gestaltet ist, dass sie von einem Abschnitt (25, 26) der leistenförmigen Erhebung (31) hintergriffen wird.

2. Körperpflegesystem (1) nach Anspruch 1, bei dem die zwei Vertiefungsseitenwände (23, 24; 29, 30) so gestaltet sind, dass sie von zwei Abschnitten (25, 26) der leistenförmigen Erhebung (31) hintergriffen werden.

3. Körperpflegesystem (1) nach Anspruch 1, bei dem die mindestens eine rillenförmige Vertiefung (21) schwalbenschwanzförmig ausgebildet ist.

4. Körperpflegesystem (1) nach Anspruch 1, bei dem mindestens eine Kombination eine ringartig in sich geschlossene rillenförmige Vertiefung (27) und eine ringartig in sich geschlossene leistenförmige Erhebung umfasst.

5. Körperpflegesystem (1) nach Anspruch 1, bei dem von der mindestens einen Kombination die mindestens eine rillenförmige Vertiefung (21; 27) in dem zweiten Abschnitt (14) der Wärmeleiteinrichtung (11) und die mindestens eine leistenförmige Erhebung (31) an dem den zweiten Abschnitt (14) der Wärmeleiteinrichtung (11) umgebenden Bereich des Gehäuses (7) vorgesehen ist.

6. Körperpflegesystem (1) nach Anspruch 1, bei dem die Wärmeleiteinrichtung (11) mindestens ein stranggepresstes Formprofilteil aus Aluminium umfasst.

7. Kühlvorrichtung (3) für ein Körperpflegesystem (1), die ein Gehäuse (7) aus Kunststoff aufweist, wobei in dem Gehäuse (7) ein Kühlmittel enthalten ist, die Kühlvorrichtung (3) eine Wärmeleiteinrichtung (11) aufweist und die Wärmeleiteinrichtung (11) einen in dem Gehäuse (7) untergebrachten und mit dem Kühlmittel in Wärmeleitverbindung stehenden ersten Abschnitt (12) und einen durch das Gehäuse (7) in einer Durchgangsrichtung (13) hindurchgehenden zweiten Abschnitt (14) und einen zum Kühlen eines zu kühlenden Bereiches eines Körpers vorgesehenen dritten Abschnitt (15) aufweist, **dadurch gekennzeichnet, dass**
zwischen dem zweiten Abschnitt (14) der Wärmeleiteinrichtung (11) und dem den zweiten Abschnitt (14) der Wärmeleiteinrichtung (11) umgebenden Bereich des Gehäuses (7) mindestens eine quer zur Durchgangsrichtung (13) verlaufende Kombination aus mindestens einer rillenförmigen Vertiefung (21) und mindestens einer leistenförmigen Erhebung (31) vorgesehen ist, und dass
die mindestens eine rillenförmige Vertiefung (21; 27) in der mindestens einen Kombination von zwei Vertiefungsseitenwänden (23, 24; 29, 30) begrenzt ist, von denen mindestens eine Vertiefungsseitenwand (23, 24; 29, 30) so gestaltet ist, dass sie von einem Abschnitt (25, 26) der leistenförmigen Erhebung (31) hintergriffen wird.

8. Kühlvorrichtung (3) nach Anspruch 7, bei der die zwei Vertiefungsseitenwände (23, 24; 29, 30) so gestaltet sind, dass sie von zwei Abschnitten (25, 26) der leistenförmigen Erhebung (31) hintergriffen werden.

9. Kühlvorrichtung (3) nach Anspruch 8, bei der die mindestens eine rillenförmige Vertiefung (21) schwalbenschwanzförmig ausgebildet ist.

10. Kühlvorrichtung (3) nach Anspruch 7, bei der mindestens eine Kombination eine ringartig in sich geschlossene rillenförmige Vertiefung (27) und eine ringartig in sich geschlossene leistenförmige Erhebung umfasst.

11. Kühlvorrichtung (3) nach Anspruch 7, bei der von der mindestens einen Kombination die mindestens eine rillenförmige Vertiefung (21; 27) in dem zweiten Abschnitt (14) der Wärmeleiteinrichtung (11) und die mindestens eine leistenförmige Erhebung (31) an dem den zweiten Abschnitt (14) der Wärmeleiteinrichtung (11) umgebenden Bereich des Gehäuses (7) vorgesehen ist.

12. Kühlvorrichtung (3) nach Anspruch 7, bei der die Wärmeleiteinrichtung (11) mindestens ein stranggepresstes Formprofilteil aus Aluminium umfasst.

## Revendications

1. Système de soins personnels (1) avec dispositif de soins personnels (2) et avec dispositif de refroidissement (3), lequel dispositif de refroidissement (3) a un logement (7) en matériau plastique, logement (7) dans lequel un agent réfrigérant est contenu, et lequel dispositif de refroidissement (3) a un dispositif conducteur de chaleur (11), le dispositif conducteur de chaleur (11) ayant une première partie (12) qui est logée dans le logement (7) et est en contact thermiquement conducteur avec l'agent réfrigérant, une deuxième partie (14) qui traverse le logement (7) dans une direction de passage (13) et une troisième partie (15) qui est destinée à refroidir une zone à refroidir d'un corps, **caractérisé en ce qu'**il est prévu entre la deuxième partie (14) du dispositif conducteur de chaleur (11) et la zone du logement (7) entourant la deuxième partie (14) du dispositif conducteur de chaleur (11) au moins une combinaison, se prolongeant transversalement à la direction de passage (13), d'au moins une dépression semblable à une gorge (21) et au moins une saillie semblable à une languette (31), et **en ce que** la au moins une dépression semblable à une gorge (21 ; 27) dans la au moins une combinaison est définie par deux parois latérales (23, 24 ; 29, 30) dont au moins une paroi latérale (23, 24 ; 29, 30) est d'une configuration telle qu'une partie (25, 26) de la saillie semblable à une languette (31) s'accroche derrière elle.

2. Système de soins personnels (1) selon la revendication 1, dans lequel la configuration des deux parois latérales (23, 24 ; 29, 30) des dépressions est telle que deux parties (25, 26) de la saillie semblable à une languette (31) s'accrochent derrière elles.

3. Système de soins personnels (1) selon la revendication 1, dans lequel au moins une dépression semblable à une gorge (21) est en forme de queue d'aronde.

4. Système de soins personnels (1) selon la revendication 1, dans lequel au moins une combinaison comprend une dépression semblable à une gorge (27) qui est refermée sur elle-même comme un anneau et une saillie semblable à une languette qui est refermée sur elle-même comme un anneau.

5. Système de soins personnels selon la revendication 1, dans lequel, dans la au moins une combinaison, la au moins une dépression semblable à une gorge (21, 27) est prévue dans la deuxième partie (14) du dispositif conducteur de chaleur (11) et la au moins une saillie semblable à une languette (31) est prévue sur la zone du logement (7) entourant la deuxième partie (14) du dispositif conducteur de chaleur (11).

6. Système de soins personnels (1) selon la revendication 1, dans lequel le dispositif conducteur de chaleur (11) comprend au moins une partie extrudée d'un profil formé fait en aluminium.

7. Dispositif de refroidissement (3) pour système de soins personnels (1), lequel dispositif de refroidissement (3) a un logement (7) en matériau plastique, logement (7) dans lequel un agent réfrigérant est contenu, le dispositif conducteur de chaleur (11) ayant une première partie (12) qui est logée dans le logement (7) et est en contact thermiquement conducteur avec l'agent réfrigérant, une deuxième partie (14) qui traverse le logement (7) dans une direction de passage (13) et une troisième partie (15) qui est destinée à refroidir une zone à refroidir d'un corps, **caractérisé en ce qu'**il est prévu entre la deuxième partie (14) du dispositif conducteur de chaleur (11) et la zone du logement (7) entourant la deuxième partie (14) du dispositif conducteur de chaleur (11) au moins une combinaison, se prolongeant transversalement à la direction de passage (13), d'au moins une dépression semblable à une gorge (21) et au moins une saillie semblable à une languette (31), et **en ce que** la au moins une dépression semblable à une gorge (21 ; 27) dans la au moins une combinaison est définie par deux parois latérales (23, 24 ; 29, 30) dont au moins une paroi latérale est d'une configuration telle qu'une partie (25, 26) de la saillie semblable à une languette (31) s'accroche derrière elle.

8. Dispositif de refroidissement (3) selon la revendication 7, dans lequel la configuration des parois latérales (23, 24 ; 29, 30) des dépressions est telle que deux parties (25, 26) de la saillie semblable à une languette (31) s'accrochent derrière elles.

9. Dispositif de refroidissement (3) selon la revendication 8, dans lequel la au moins une dépression semblable à une gorge (21) est en forme de queue d'aronde.

10. Dispositif de refroidissement (3) selon la revendication 7, dans lequel au moins une combinaison comprend une dépression semblable à une gorge (27) qui est refermée sur elle-même comme un anneau et une saillie semblable à une languette (31) qui est refermée sur elle-même comme un anneau.

11. Dispositif de refroidissement (3) selon la revendication 7, dans lequel, dans la au moins une combinaison, la au moins une dépression semblable à une gorge (21 ; 27) est prévue dans la deuxième partie (14) du dispositif conducteur de chaleur (11) et la au moins une saillie semblable à une languette (31) est prévue sur la zone du logement (7) entourant la deuxième partie (14) du dispositif conducteur de chaleur (11).

12. Dispositif de refroidissement (3) selon la revendication 7, dans lequel le dispositif conducteur de chaleur (11) comprend au moins une partie extrudée d'un profil formé fait en aluminium.
